# EUROPÄISCHE PATENTSCHRIFT

(11) **EP 0 562 583 B1**
(45) Veröffentlichungstag und Bekanntmachung des Hinweises auf die Patenterteilung: **16.04.1997**
(21) Anmeldenummer: 93104890.4
(22) Anmeldetag: 24.03.1993
(51) Int. Cl.: C07D 503/18

(54) **Neue Alkylendiammonium-diclavulanat-Derivate, Verfahren zu deren Herstellung sowie deren Verwendung**
Novel alkylene diammonium diclavulanate derivatives, process for their preparation and for their use
Nouveaux dérivés d'alkylènediammonium diclavulanates, procédés pour leur préparation et pour leur utilisation

(30) Priorität: 26.03.1992 AT 619/92
(43) Veröffentlichungstag der Anmeldung: 29.09.1993
(73) Patentinhaber: LEK, tovarna farmacevtskih in kemicnih izdelkov, d.d., SI-61107 Ljubljana (SI)
(72) Erfinder: Copar, Anton, Dr. Ing., 61275 Smartno pri Litiji (SL)
(74) Vertreter: Perrey, Ralf

(56) Entgegenhaltungen:
- EP-A- 0 002 312
- EP-A- 0 026 044
- EP-A- 0 387 178
- DE-A- 2 708 046
- FR-A- 1 290 132
- DATABASE WPIL Week 8649, Derwent Publications Ltd., London, GB; AN 86-320905 & ES-A-8 606 492

## Beschreibung

Die vorliegenden Erfindung gehört ins Gebiet der pharmazeutischen Industrie und betrifft neue Alkylendiammonium-diclavulanat-Derivate, Verfahren zu deren Herstellung sowie deren Verwendung als Zwischenverbindungen zur Herstellung von reiner Clavulansäure und deren pharmazeutisch annehmbaren Alkalisalzen wie Kalium-clavulanat.

Clavulansäure, (2R,5R,Z)-3-(2-Hydroxyethyliden)-7-oxo-4-oxa-1-azabicyclo-[3,2,0]-heptan-2-carbonsäure, ist eine bereits bekannte Verbindung der folgenden Struktur: 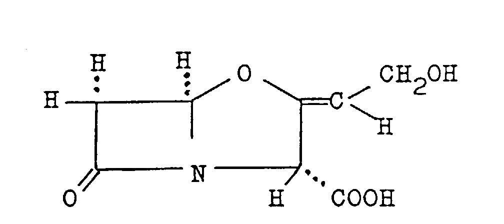 Diese Verbindung sowie ihre Salze und Ester wirken als β-Lactamase-Hemmer, d.h. sie hemmen die durch Gram-positive und Gram-negative Mikroorganismen gebildeten β-Lactamasen. Clavulansäure bzw. deren Salze werden deswegen in galenischen Präparaten verwendet, um die Desaktivierung der β-Lactam-Antibiotika zu verhindern. Kommerzielle Präparate enthalten ein stabileres Kaliumsalz der Clavulansäure (die Clavulansäure ist an sich ziemlich unstabil), kombiniert mit Amoxycyllintrihydrat.

Clavulansäure wird auf fermentativem Weg aus verschiedenen Mikroorganismen gewonnen, die zu verschiedenen Stämmen von Streptomyceten, wie S. clavuligerus NRRL 3585, S. jumoninensis NRRL 5741, S. katsurahamanus IFO 13716 und Streptomyces sp. P 6621 FERM P2804, gehören.

Clavulansäure und deren Salze wurden zuerst in GB 1 508 977 beschrieben. Das dort beschriebene Verfahren zur Herstellung von Clavulansäure ist zeitraubend und beruht auf anspruchsvollen Reinigungen mittels verschiedener chromatographischer Methoden. Die Salze der Clavulansäure werden durch das Binden des im Filtrat der Fermentationsbrühe anwesenden Clavulanat-Anions an ein Anionenaustauscherharz, dessen Elution daraus durch einen Elektrolyten, die Entsalzung des erhaltenen Eluats, die Aufgabe des letzteren auf ein anderes Anionenaustauscherharz, anschliessende chromatographische Elution daraus durch einen Elektrolyten, eine erneute Entsalzung des erhaltenen Eluats und das Entfernen des Lösungsmittels gewonnen.

Das Verfahren erfordert die Verwendung von präparativen Chromatographiesäulen, was einen erheblichen Investitionsaufwand bedeutet, und ausserdem ist die Verwendbarkeit im grosstechnischen Masstab beschränkt.

Ein weiterer Nachteil dieses Verfahrens liegt darin, dass die meisten Verfahrensschritte in einem wässrigen Medium, in dem Clavulansäure sehr unbeständig ist, durchgeführt werden.

In GB 1 543 563 wird ein modifiziertes fermentatives Verfahren beschrieben, bei dem der pH-Wert des Mediums innerhalb der Grenzen von 6,3 bis 6,7 aufrechterhalten wird, wodurch die Ausbeute an der gewünschten Verbindung erhöht wird. Die Clavulansäure-Salze wie Kaliumclavulanat werden durch eine doppelte Umsetzung aus Lithiumclavulanat gewonnen.

Gemäss dem in EP-0-026044 beschriebenen Verfahren zur Herstellung von Clavulansäure und deren pharmazeutisch annehmbaren Salzen werden die anspruchsvollen Methoden der Reinigung mittels Austauscherharze weitgehend vermieden. Dem Verfahren liegt die Herstellung des tert.-Butylaminsalzes der Clavulansäure, vorzugsweise in der Form des Aceton-Solvats, zugrunde. Das tert.-Butylaminsalz der Clavulansäure wird seinerseits durch die Behandlung des rohe Clavulansäure enthaltenden Extrakts, vorzugsweise des Ethylacetatextrakts, das gemäss dem in GB 1 508 977 beschriebenen Verfahren hergestellt wurde, mit tert.-Butylamin in einem organischen Lösungsmittel wie Aceton und anschliessende Umsetzung des isolierten tert.-Butylaminsalzes der Clavulansäure zu Clavulansäure bzw. zu deren pharmazeutisch annehmbarem Salz gewonnen.

DE-A-27 08 046 betrifft ein Verfahren zur Herstellung von kristallinem Calcium-diclavulanat-dihydrat und diese Verbindung enthaltende Arzneimittel. Dazu wird eine wäßrige Lösung eines anderen Salzes der Clavulansäure als das Calciumsalz mit einem Kationenaustauscherharz in Form des Calciumsalzes in Berührung gebracht, aus dem Austauscherharz das gebildete Calciumdiclavulanat eluiert und eine von anderen Kationen als Calciumionen praktisch freie Lösung des Calcium-di-clavulanat erhalten, aus der man anschließend das Calcium-di-clavulanat auskristallisieren läßt.

EP-A-0 002 312 beschreibt die Herstellung von Derivaten der Clavulansäure, wobei als Zwischenprodukte Tertiärbutylaminsalze gebildet werden.

Database WPIL, Week 8649, AN-86-320905 beschreibt die Herstellung von Alkalimetallsalzen der Clavulansäure, wobei ein quaternäres Ammoniumsalz der Clavulansäure gebildet wird und dieses mit einem Alkali- oder Erdalkalimetallthiocyanat in Aceton reagiert.

EP-A-0 387 178 beschreibt ein Verfahren zum Erhalt von Z(2R,5R)-3-(2-hydroxy-ethyliden)-7-oxo-4-oxa-1-azabicyclo(3,2,0)-heptan-2-carbonsäure(= Clavulansäure) und seiner pharmazeutisch verträglichen Salze aus Fermentationsbrühen von Streptomyces sp., wobei ein zentraler Schritt der Erhalt von Benzylterbutylaminclavulanat ist.

FR-A-1 290 132 beschreibt die Verwendung von N,N'-Dibenzyl-ethylen-diamin zur Herstellung von Benzatin-Penicillin.

Der Erfindung liegt die Aufgabe zugrunde, reine Clavulansäure und deren pharmazeutisch annehmbare Salze wie ihr Kaliumsalz auf eine neue und einfache Weise herzustellen, bei der man die gewünschte Substanz mit einer hohen Ausbeute und einer hohen Reinheit durch Isolation aus dem Konzentrat des auf bekannte Weisen gewonnenen Extraktes aus einem organischen Lösungsmittel, nach der Fermentation mit einem Clavulansäure produzierenden Mikroorganismus, worin die Clavulansäure in roher Form vorliegt, erhalten würde.

Diese Aufgabe wird dadurch gelöst, dass man die nach der Fermentation mit einem Clavulansäure produzierenden Mikroorganismus der Gattung Streptomyces sp. P 6621 FERM P2804, wie er in JP Kokai 80-162993 beschrieben wird, gewonnene Fermentationsbrühe vor der Solventextration, die eine Lösung der rohen Clavulansäure in einem organischen Lösungsmittel liefert, auf konventionelle Weise behandelt.

Für die Extraktion geeignete organische Lösungsmittel sind Ethylacetat, Methylisobutylketon, Butanol oder das gemisch von Ethylacetat mit Aceton, Ethylmethylketon oder Methylisobutylketon, vorzugsweise im verhältnis von 1:0,5 bis 1:5, doch wird ganz bevorzugt Ethylacetat verwendet.

Die organische Phase wird anschliessend mit Wasser gewaschen und durch Eindampfen eingeengt, wobei eine Konzentration an roher Clavulansäure von mindestens 20 g/l und ein Restgehalt an Wasser unter 6 g/l erreicht werden. Der erhaltene Extrakt im organischen Lösungsmittel, wie Ethylacetatextrakt, wird noch einer Behandlung mit aktiver Kohle unterzogen, um die farbigen Substanzen zu entfernen.

Der auf die oben beschriebene Weise hergestellte Extrakt wird mit substituierten Alkylendiaminen der Formel II 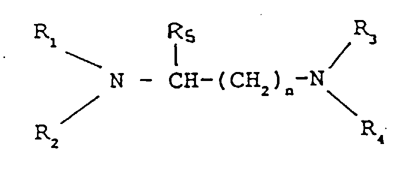 worin die Substituenten R₁, R₂, R₃ und R₄
- ein Wasserstoffatom,
- eine geradkettige oder verzweigtkettige, 1 bis 8 Kohlenstoffatome enthaltende Alkyl-Gruppe oder
- eine 2 bis 4 Kohlenstoffatome enthaltende Hydroxyalkylgruppe
bedeuten und NR₁R₂ und/oder NR₃R₄ zusammen einen heterocyclischen Ring mit 3 bis 6 an das Stickstoffatom gebundenen Methylengruppen bedeuten, wobei eine dieser Gruppen gegebenenfalls durch ein Sauerstoff- oder Schwefelatom oder eine Iminogruppe substituiert ist, und
R₅ ein Wasserstoffatom oder eine Methylgruppe und
n eine ganze Zahl 1 bis 3 bedeuten,
umgesetzt,
wobei Alkylendiammonium-diclavulanate der Formel I 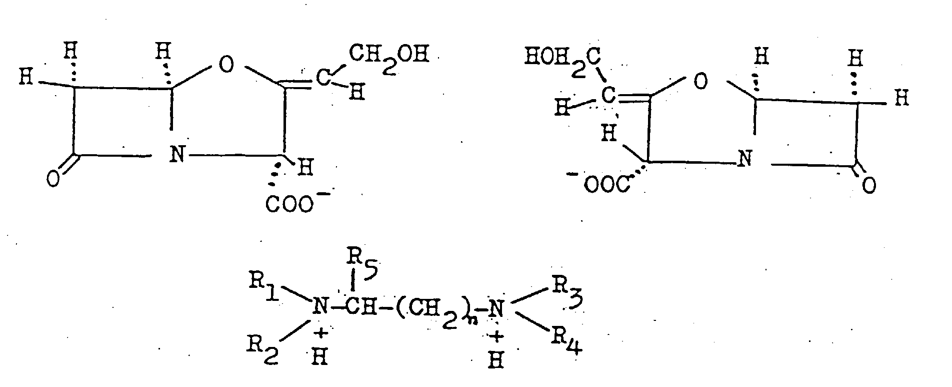 worin die Substituenten die oben angegebenen Bedeutungen besitzen, erhalten werden. Diese Salze sind neu und in der Literatur noch nicht beschrieben.

Die Umsetzungen verlaufen am günstigsten unter Verwendung von N,N'-monosubstituierten symmetrischen Ethylendiaminen, die eine Alkylkette mittlerer Länge aufweisen, worin R₁ und R₃ eine Ethyl- oder iso-Propylgruppe, R₂ und R₄ einen Wasserstoffatom und n 1 bedeuten.

Als besonders bevorzugtes Alkylendiamin wird N,N'-Diisopropylethylendiamin verwendet, das N,N'-Diisopropyl-ethylendiammonium-diclavulanat ergibt.

Die eingesetzten Alkylendiaminbasen können entweder als solche oder - falls sie in fester Form vorliegen - als Lösungen in organischen Lösungsmitteln, wie Aceton oder Ethylacetat, verwendet werden.

Zur Herstellung des Salzes der Clavulansäure wird mindestens ein Äquivalent des gewählten Alkylendiamins verwendet.

Die gewünschten Salze der Clavulansäure (1:2) der Formel I, wie N,N'-Diisopropyl-ethylendiammonium-diclavulanat, können dann isoliert werden, wodurch die Clavulansäure von den meisten oder sogar allen Verunreinigungen befreit wird.

Die gewünschten Salze der Clavulansäure der Formel I sind stabile kristalline Salze, die sich durch eine hohe Reinheit auszeichnen, so dass deren zusätzliche Reinigung durch die Umkristallisation nicht notwendig ist.

Sie werden als Zwischenverbindungen zur Herstellung von reiner Clavulansäure und deren pharmazeutisch annehmbaren Salzen, z.B. zur Herstellung von Kalium-clavulanat, verwendet, wobei sie einer doppelten Umsetzung mit einem Karbonat, Hydrogenkarbonat oder Hydroxyd eines pharmazeutisch annehmbaren Alkalimetalls oder mit einem Salz der entsprechenden Alkansäure wie Kalium-2-ethylhexanoat, in einem organischen Lösungsmittel wie Isopropanol unterzogen werden. Bei dieser Umsetzung findet der Ionenaustausch des Alkylendiammoniumkations gegen das Alkalimetallkation statt.

Ein weiterer Gegenstand der vorliegenden Erfindung ist ein Verfahren zur Reinigung der Clavulansäure und deren pharmazeutisch annehmbarer Salze, das dadurch gekennzeichnet ist, dass man
- rohen Clavulansäure extrakt in einem organischen Lösungsmittel, der sich aus der Solventextraktion der nach der Fermentation mit einem Clavulansäure produzierenden Mikroorganismus gewonnenen Fermentationsbrühe nach derenüblicher Verarbeitung ergibt, mit einem entsprechenden substituierten Alkylendiamin der Formel II 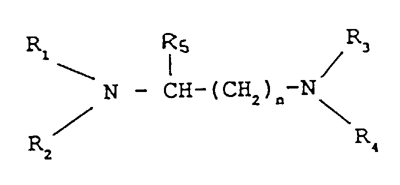 worin die Substituenten R₁, R₂, R₃, R₄ und R₅ und n die eingangs genannten Bedeutungen besitzen, umsetzt,
- das erhaltene Alkylendiammmonium-clavulanat -clavulanat der Formel I, 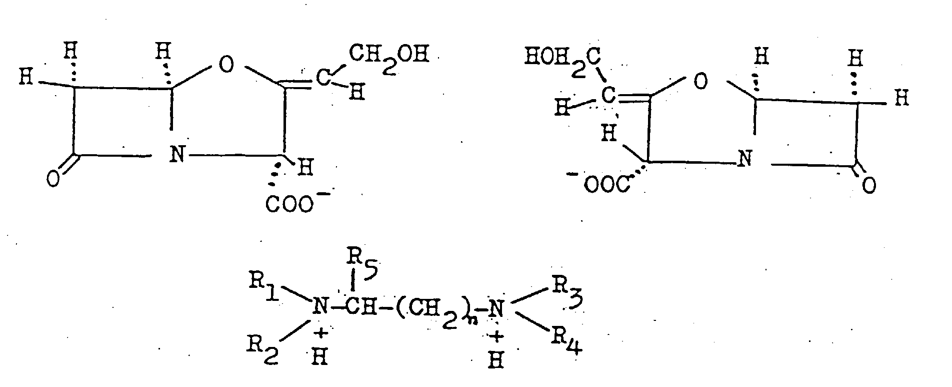 worin die Substituenten R₁, R₂, R₃, R₄ und R₅ und n die eingangs genannten Bedeutungen besitzen, gegebenenfalls isoliert und
- anschliessend zu reiner Clavulansäure oder deren pharmazeutisch annehmbaren Salzen umsetzt.

Alle erfindungsmässigen Verfahrensschritte werden bei Temperaturen um die Raumtemperatur durchgeführt.

Gegenüber der bekannten Methode zur Herstellung von Clavulansäure und deren pharmazeutisch annehmbaren Salzen, wie sie in EP-0-026 044 beschrieben wird, zeichnet sich das erfindungsmässige Verfahren durch die Herstellung des Zwischenproduktes, d.h. des Salzes der Formel I in hoher Ausbeute und Reinheit ohne Notwendigkeit einer zusätzlichen Reinigung durch Umkristallisation aus, wobei dieses Zwischenprodukt direkt aus dem Etylacetatextrakt der Clavulansäure ohne Verwendung weiterer organischer Lösungsmittel wie Aceton gewonnen werden kann. Gemäss dem in EP-0-026044 beschriebenen Verfahren wird das Clavulanat durch tert.-Butylamin gefällt, der erhaltene Niederschlag wird mit Aceton behandelt, durch Umfällung mit Aceton aus der isopropanolischen Lösung gereinigt und anschliessend in einer isopropanolischen Lösung zum Kaliumsalz der Clavulansäure umgesetzt

Die Erfindung wird durch die folgenden, in keiner Weise einschränkenden Beispiele näher erläutert.

### HERSTELLUNG VON AUSGANGSSTOFFEN

### Beispiel 1

### N,N'-Diisopropylethylendiammonium-diclavulanat

1 l des nach bekannten Methoden hergestellten Ethylacetatextraktes (Gehalt an roher Clavulansäure 20 g/l, Gehalt an Wasser 6 g/l), der vorher durch Behandlung mit aktiver Kohle teilweise entfärbt wurde, wurde unter starkem Rühren innerhalb von 10 Minuten mit 9 ml N,N'-Diisopropylethylendiamin versetzt, und die Lösung wurde noch 30 min weitergerührt, wobei ein Niederschlag ausfiel. Der erhaltene Niederschlag würde in 20 ml Wasser, das mit 400 ml Aceton verdünnt wurde, gelöst, wobei 13,0 g N,N'-Diisopropylethylendiammonium-diclavulanat in Form feiner Kristalle, Smp. 130-132 °C, ausschieden.
- ¹H-NMR (D₂O, DSS, 300 Hz):: δ = 1,33 (6H, d, J=6,5 Hz, CH(CH₃)₂), 3,12 (1H, d, J=17,0 Hz, 6-βCH), 3,38 (2H, s, NCH₂), 3,45 (1H, hept, J=6,5 Hz, CHMe₂), 3,55 (1H, dd, J=17,0 and 2,8Hz, 6-αCH), 4,18 (1H, d, J=7,4 Hz, CH', H"OH), 4,19 (1H, d, J=7,8 Hz, CH'H''OH), 4,88-4,96 (2H, m, 3-CH, =CH-), 5,72 (1H, d, J=2,8 Hz).

### Beispiel 2

### N,N'-Diethylethylendiammonium-diclavulanat

1 l des nach bekannten Methoden hergestellten Ethylacetatextraktes (Gehalt an roher Clavulansäure 20 g/l, Gehalt an Wasser 6 g/l), der vorher durch Behandlung mit aktiver Kohle teilweise entfärbt wurde, wurde mit 2 1 Aceton und anschliessend unter starkem Rühren innerhalb von 15 Minuten noch mit 6,4 ml N,N'-Diethylethylendiamin versetzt. Das erhaltene Gemisch wurde noch 30 min weitergerührt, der ausgeschiedene Niederschlag wurde abgefiltert und mit Aceton gewaschen. Der erhaltene amorphe Niederschlag wurde in 1 1 Aceton suspendiert, durch starkes Rühren zerschlagen, und die Suspension wurde nochmals filtriert. Das erhaltene Produkt wurde getrocknet, in 20 ml Wasser gelöst, und die Lösung wurde mit 200 ml Aceton versetzt. Der klare Anteil des Gemisches wurde von der weichen, an den Wänden des Gefässes anhaftenden Masse dekantiert und filtriert. Das Filtrat wurde nochmals mit 400 ml Aceton versetzt, wobei sich ein Niederschlag bildete, der abgefiltert und mit Aceton gewaschen wurde. Es wurden 10 g der gewünschten Verbindung, Smp. 104-108 °C, erhalten.
- ¹H-NMR (D₂O, DSS, 300 Hz):: δ = 1,29 (3H, t, J=7,3 Hz, CH₂CH₃), 3,11 (1H, d, J=17,0 Hz, 6-βCH), 3,16 (2H, q, J=7,3 Hz, CH₂CH₃), 3,41 (2H, s, NCH₂), 3,55 (1H, dd, J=17,0 und 2,7 Hz, 6-αCH), 4,18 (1H, d, J=8,2 Hz, CH', H''OH), 4,18 (1H, d, J=8,0 Hz, CH'H''OH), 5,85-5,96 (2H, m, 3-CH, =CH-), 5,72 (1H, d, J=2,7 Hz).

### DAS ERFINDUNGSMÄSSE VERFAHREN

### Beispiel 3

### Kalium-clavulanat

10 g N,N'-Diisopropylethylendiammonium-diclavulanat (Gehalt an Clavulansäure 69%) wurden in 10 ml Wasser gelöst, die Lösung wurde mit 190 ml Isopropanol verdünnt und unter Rühren innerhalb von 15 min mit 15 ml einer 2M Lösung von Kalium-2-ethylhexanoat in Isopropanol versetzt. Die erhaltene Suspension wurde noch 30 min gerührt, wobei ein Niederschlag ausfiel, der abgefiltert, mit Isopropanol gewaschen und anschliessend getrocknet wurde. Es wurden 4,8 g (70%) Kalium-clavulanat (USP Grade, Gehalt an Clavulansäure 81%, ermittelt nach der HPLC-Methode) erhalten.

### Beispiel 4

### Kalium-clavulanat

10 g N,N'-Diethylethylendiammonium-diclavulanat (Gehalt an Clavulansäure 70%) wurden in 10 ml Wasser gelöst, und die Lösung wurde mit 190 ml Isopropanol versetzt. Das Gemisch wurde filtriert, und das Filtrat wurde unter Rühren innerhalb von 15 min mit 15 ml einer 2M Lösung von Kalium-2-ethylhexanoat in Isopropanol versetzt. Die erhaltene Suspension wurde noch 30 min gerührt, wobei sich ein Niederschlag bildete, der abgefiltert, mit Isopropanol gewaschen und anschliessend getrocknet wurde. Es wurden 4,0 g (47%) Kalium-clavulanat (USP Grade, Gehalt an Clavulansäure 81%, ermittelt nach der HPLC-Methode) erhalten.

## Patentansprüche

1. Alkylendiammonium-diclavulanate der Formel I 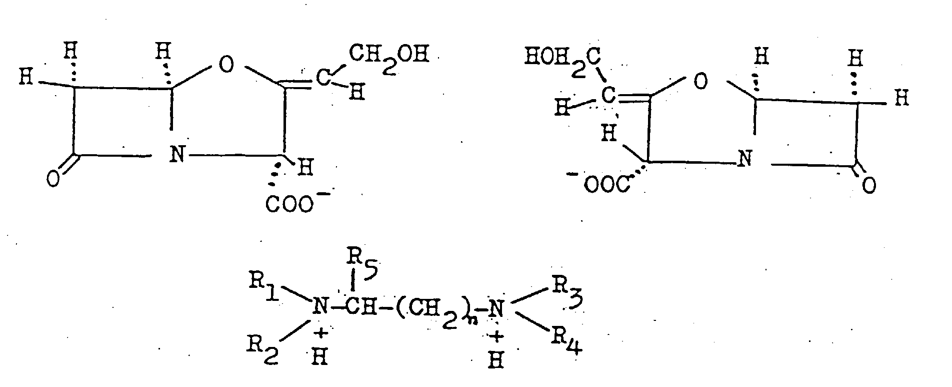 worin die Substituenten R₁, R₂, R₃ und R₄
- ein Wasserstoffatom,
- eine geradkettige oder verzweigtkettige, 1 bis 8 Kohlenstoffatome enthaltende Alkyl-Gruppe oder
- eine 2 bis 4 Kohlenstoffatome enthaltende Hydroxyalkylgruppe bedeuten und NR₁R₂ und/oder NR₃R₄ zusammen einen heterocyclischen Ring mit 3 bis 6 an das Stickstoffatom gebundenen Methylengruppen bedeuten, wobei eine dieser Gruppen gegebenenfalls durch ein Sauerstoff- oder Schwefelatom oder eine Iminogruppe substituiert ist, und
R₅ ein Wasserstoffatom oder eine Methylgruppe und
n eine ganze Zahl von 1 bis 3 bedeuten.

2. N,N'-Diisopropylethylendiammonium-diclavulanat.

3. N,N'-Diethylethylendiammonium-diclavulanat.

4. Verfahren zur Herstellung von Verbindungen der Formel I nach Anspruch 1, dadurch gekennzeichnet, daß man rohen Clavulansäureextrakt in einem organischen Lösungsmittel, der sich aus Solventextraktion der nach der Fermentation mit einem Clavulansäure-produzierenden Mikroorganismus gewonnenen Fermentationsbrühe nach deren üblicher Verarbeitung ergibt, mit substituierten Alkylendiaminen der Formel II 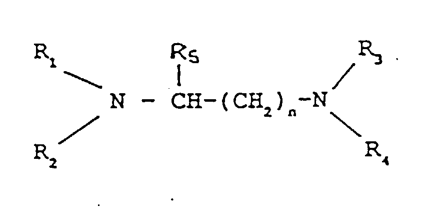 worin die Substituenten R₁, R₂, R₃, R₄ und R₅ sowie n die unter der Formel I im Anspruch 1 genannten Bedeutungen besitzen, gegebenenfalls mit deren Lösungen in einem organischen Lösungsmittel, wie Aceton oder Ethylacetat, umsetzt.

5. Verfahren nach Anspruch 4, dadurch gekennzeichnet, daß man als organisches Lösungsmittel Ethylacetat, Methylisobutylketon, Butanol oder das gemisch von Ethylacetat mit Aceton, Ethylmethylketon oder Methylisobutylketon verwendet.

6. Verfahren zur Herstellung des N,N'-Diisopropylethylendiammoniumdiclavulanats nach Anspruch 2, dadurch gekennzeichnet, daß man rohen Clavulansäureextrakt in Ethylacetat mit N,N'-Diisopropylethylendiamin umsetzt und das dabei erhaltene N,N'-Diisopropylethylendiammoniumdiclavulanatgegebenenfalls isoliert.

7. Verwendung von Alkylendiammonium-diclavulanaten der Formel I nach einem der Ansprüche 1 bis 3 als Zwischenverbindungen bei der Herstellung von reiner Clavulansäure und deren pharmazeutisch annehmbaren Alkalisalzen.

8. Verwendung des N,N'-Diisopropylethylendiammoniumdiclavulanats nach Anspruch 2 als Zwischenverbindung bei der Herstellung von reinem Kaliumclavulanat.

9. Verfahren zur Herstellung von reiner Clavulansäure und deren pharmazeutisch annehmbaren Alkalisalzen, dadurch gekennzeichnet, daß man Alkylendiammoniumdiclavulanate der Formel I nach einem der Ansprüche 1 bis 3 zu reiner Clavulansäure oder deren pharmazeutisch annehmbaren Alkalisalzen umsetzt.

10. Verfahren zur Herstellung von reinem Kaliumclavulanat, dadurch gekennzeichnet, daß man N,N'-Diisopropylethylendiammoniumdiclavulanat nach Anspruch 2 zu reinem Kaliumclavulanat umsetzt.

11. Verfahren zur Herstellung von reiner Clavulansäure und deren pharmazeutisch annehmbaren Alkalisalzen, dadurch gekennzeichnet, daß man
a) rohen Clavulansäureextrakt in einem organischen Lösungsmittel, der sich aus Solventextraktion der nach der Fementation mit einem Clavulansäure produzierenden Mikroorganismus gewonnenen Fermentationsbrühe nach deren üblicher Verarbeitung ergibt, mit einem substituierten Alkylendiamin der Formel II 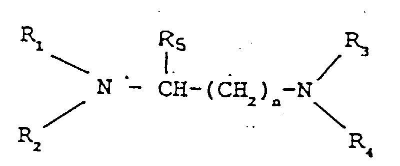 worin die Substituenten R₁, R₂, R₃, R₄ und R₅ sowie n die unter der Formel I im Anspruch 1 genannten Bedeutungen besitzen, umsetzt,
b) das dabei erhaltene Alkylendiammonium-diclavulanat der Formel I 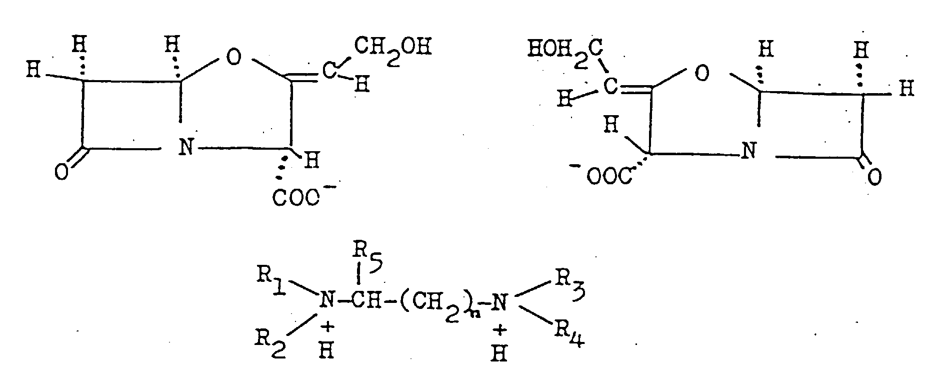 worin die Substituenten R₁, R₂, R₃, R₄ und R₅ sowie n die unter der Formel I im Anspruch 1 genannten Bedeutungen besitzen, isoliert und
c) anschließend zu reiner Clavulansäure oder deren pharmazeutisch annehmbaren Alkalisalzen umsetzt.

12. Verfahren nach einem der Ansprüche 9 oder 11, dadurch gekennzeichnet, daß man das reine Kaliumclavulanat durch Umsetzung von Alkylendiammonium-diclavulanat der Formel I mit Kalium-2-ethyl-hexanoat in Isopropanol herstellt.

13. Verfahren nach einem der Ansprüche 9, 11 oder 12, dadurch gekennzeichnet, daß man das organische Lös ngsmittel aus der Gruppe bestehend aus Ethylacetat, Methylisobutylketon, Butanol oder einem Gemish von Ethylacetat mit Aceton, Ethylmethylketon oder Methylisobutylketon auswählt.

14. Verfahren nach Anspruch 13, dadurch gekennzeichnet, daß man als organisches Lösungsmittel Ethylacetat verwendet.

15. Verfahren zur Herstellung von reinem Kaliumclavulanat, dadurch gekennzeichnet, daß man
a) rohen Clavulansäureextraktin Ethylacetat mit N,N'-Diisopropylethylendiamin umsetzt,
b) das dabei erhaltene N,N'-Diisopropylethylendiammoniumdiclavulanatisoliert und
c) anschließend mit Kalium-2-ethylhexanoat in Wasser enthaltendem Isopropanol zu reinem Kaliumclavulanat umsetzt und das gewünschte Salz isoliert.

## Claims

1. Alkylenediammonium diclavulanates of the formula I 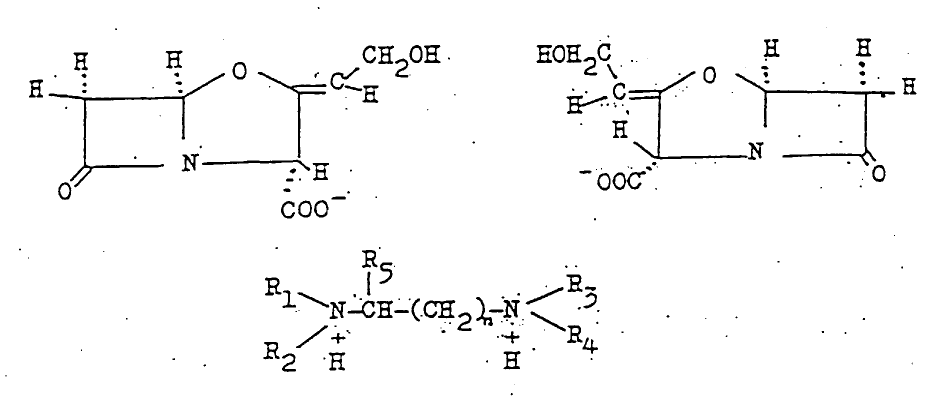 wherein the substituents R₁, R₂, R₃ and R₄ denote
- a hydrogen atom,
- a straight-chain or branched-chain alkyl group having 1 to 8 carbon atoms or
- a hydroxylakyl group having 2 to 4 carbon atoms and NR₁R₂ and/or NR₃R₄ jointly denote a heterocyclic ring having 3 to 6 methylene groups bound to a nitrogen atom, one of these groups being optionally substituted by an oxygen or a sulphur atom or by an imino group and
R₅ denotes a hydrogen atom, or a methyl group and
n denotes an integer from 1 to 3.

2. N,N'-diisopropylethylenediammonium diclavulanate.

3. N,N'-diethylethylenediammonium diclavulanate.

4. A process for the preparation of compounds of the formula I, according to claim 1, characterized in that a crude clavulanic extract in an organic solvent resulting from a solvent extraction of the fermentation broth obtained after the common processing thereof after the fermentation with a microorganism, producer of clavulanic acid, is reacted with substituted alkylenediamines of the formula II 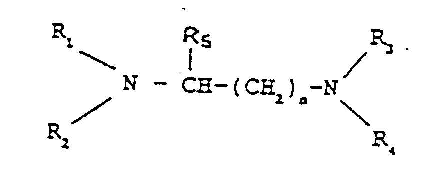 wherein the substituents R₁, R₂, R₃, R₄ and R₅ and n have the meanings as defined at formula I in claim 1, optionally with solutions thereof in an organic solvent, such as acetone or ethyl acetate.

5. A process according to claim 4, characterized in that ethyl acetate, methyl isobutyl ketone, butanol or a mixture of ethyl acetate with acetone, ethyl methyl ketone or methyl isobutyl ketone is used as the organic solvent.

6. A process for the preparation of N,N'-diisopropylethylenediammonium diclavulanate according to claim 2, characterized in that a crude clavulanic acid extract is reacted in ethyl acetate with N,N'-diisopropylethylenediamine and the obtained N,N'-diisopropylethylenediammonium diclavulanate is optionally isolated.

7. Use of the alkylenediammonium diclavulanates of the formula I according to one of the claims 1 to 3 as intermediate compounds in the preparation of pure clavulanic acid and of pharmaceutically acceptable alkali salts thereof.

8. Use of N,N'-diisopropylethylenediammonium diclavulanate according to claim 2 as the intermediate compound in the preparation of pure potassium clavulanate.

9. A process for the preparation of pure clavulanic acid and of pharmaceutically acceptable alkali salts thereof, characterized in that the alkylenediammonium diclavulanates of the formula I according to one of the claims 1 to 3 are reacted to pure clavulanic acid or pharmaceutically acceptable alkali salts thereof.

10. A process for the preparation of pure potassium clavulanate, characterized in that N,N'-diisopropylethylenediammonium diclavulanate according to claim 2 is reacted to pure potassium clavulanate.

11. A process for the preparation of pure clavulanic acid and of pharmaceutically acceptable alkali salts thereof, characterized in that
a) a crude clavulanic extract in an organic solvent resulting from solvent extraction of the fermentation broth obtained after the common processing thereof after the fermentation with a microorganism, producer of clavulanic acid, is reacted with a substituted alkylenediamine of the formula II 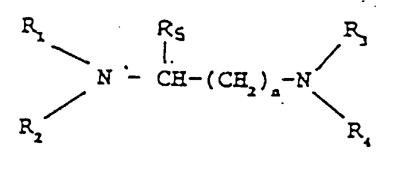 wherein the substituents R₁, R_{2,} R₃, R₄ and R₅ and n have the meanings as defined at formula I in claim 1,
b) the obtained alkylenediammonium diclavulanate of the formula I 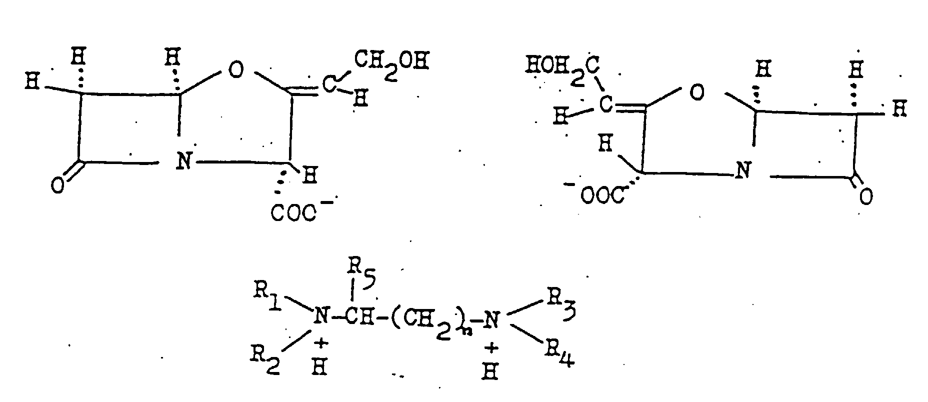 wherein the substituents R₁, R_{2,} R₃, R₄ and R₅ and n have the meanings as defined at formula I in claim 1, is isolated and
c) subsequently converted to pure clavulanic acid or pharmaceutically acceptable alkali salts thereof.

12. A process according to one of the claims 9 or 11, characterized in that the pure potassium clavulanate is prepared by reacting alkylenediammonium diclavulanate of the formula I with potassium 2-ethylhexanoate in isopropanol.

13. A process according to one of the claims 9, 11 or 12, characterized in that the organic solvent is selected from the group comprising ethyl acetate, methyl isobutyl ketone, butanol or a mixture of ethyl acetate with acetone, ethyl methyl ketone, or methyl isobutyl ketone.

14. A process according to claim 13, characterized in that ethyl acetate is used as the organic solvent.

15. A process for the preparation of pure potassium clavulanate, characterized in that
a) crude clavulanic acid extract is reacted with N,N'-diisopropylethylenediamine in ethyl acetate,
b) the obtained N,N'-diisopropylethylenediammonium diclavulanate is isolated and
c) subsequently reacted with potassium 2-ethylhexanoate in aqueous isopropanol to pure potassium clavulanate and the desired salt is isolated.

## Revendications

1. Diclavulanates d'alkylène diammonium de formule I 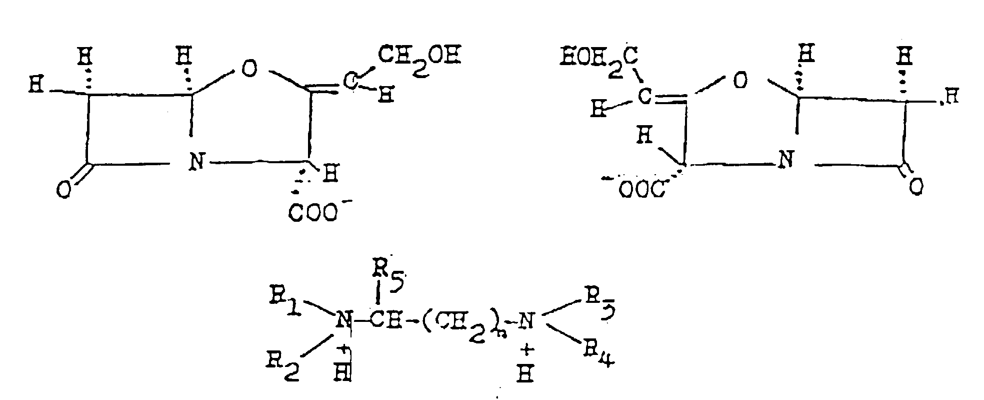 dans laquelle les substituants R₁, R₂, R₃ et R₄ représentent
- un atome d'hydrogène,
- un groupe alkyle à chaîne droite ou ramifiée contenant de 1 à 8 atomes de carbone, ou
- un groupe hydroxyalkyle contenant de 2 à 4 atomes de carbone, et
NR₁R₂ et/ou NR₃R₄ représentent ensemble un composé hétérocyclique ayant de 3 à 6 groupes méthylène liés à l'atome d'azote, l'un de ces groupes étant le cas échéant substitué par un atome d'oxygène ou de soufre ou bien par un groupe imino, et
R₅ représente un atome d'hydrogène ou un groupe méthyle et
n est un nombre entier compris entre 1 et 3.

2. Diclavulanate de N,N'-diisopropyléthylène diammonium.

3. Diclavulanate de N,N'-diéthyléthylène diammonium.

4. Procédé de fabrication de composés de formule I selon la revendication 1, caractérisé en ce que l'on fait réagir de l'extrait d'acide clavulanique brut dans un solvant organique, tiré par extraction de solvant du bouillon de fermentation obtenu à la suite de la fermentation avec un micro-organisme produisant de l'acide clavulanique après traitement usuel de ce bouillon, avec des alkylène diamines substituées de formule II 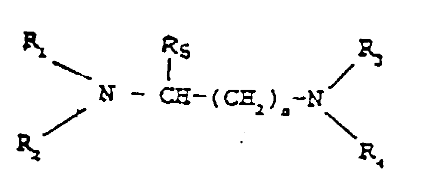 dans laquelle les substituants R₁, R₂, R₃, R₄ et R₅ ainsi que n ont les significations indiquées pour la formule I dans la revendication 1, le cas échéant avec les solutions de celles-ci dans un solvant organique tel que l'acétone ou l'acétate d'éthyle.

5. Procédé selon la revendication 4, caractérisé en ce que le solvant organique utilisé est l'acétate d'éthyle, la méthylisobutylcétone, le butanol ou un mélange d'acétate d'éthyle et d'acétone, d'éthylméthylcétone ou de méthylisobutylcétone.

6. Procédé de fabrication du diclavulanate de N,N'-diisopropyléthylène diammonium selon la revendication 2, caractérisé en ce que l'on fait réagir de l'extrait d'acide clavulanique brut dans de l'acétate d'éthyle avec de la N,N'-diisopropyléthylène diamine et en ce que le diclavulanate de N,N'-diisopropyléthylène diammonium ainsi obtenu est le cas échéant isolé.

7. Utilisation de diclavulanates d'alkylène diammonium de formule I selon l'une quelconque des revendications 1 à 3 comme composés intermédiaires dans la fabrication d'acide clavulanique pur et des sels alcalins pharmaceutiquement acceptables de celui-ci.

8. Utilisation du diclavulanate de N,N'-diisopropyléthylène diammonium selon la revendication 2 comme composé intermédiaire dans la fabrication de clavulanate de potassium pur.

9. Procédé de fabrication d'acide clavulanique pur et des sels alcalins pharmaceutiquement acceptables de celui-ci, caractérisé en ce que l'on convertit des diclavulanates d'alkylène diammonium de formule I selon l'une quelconque des revendications 1 à 3 en acide clavulanique pur ou en des sels alcalins pharmaceutiquement acceptables de celui-ci.

10. Procédé de fabrication de clavulanate de potassium pur, caractérisé en ce que l'on convertit du diclavulanate de N,N'-diisopropyléthylène diammonium selon la revendication 2 en clavulanate de potassium pur.

11. Procédé de fabrication d'acide clavulanique pur et des sels alcalins pharmaceutiquement acceptables de celui-ci, caractérisé en ce que
a) on fait réagir de l'extrait d'acide clavulanique brut dans un solvant organique, tiré par extraction de solvant du bouillon de fermentation obtenu à la suite de la fermentation avec un micro-organisme produisant de l'acide clavulanique après traitement usuel de ce bouillon, avec une alkylène diamine substituée de formule II 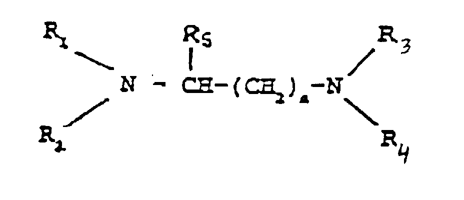 dans laquelle les substituants R₁, R₂, R₃, R₄ et R₅ ainsi que n ont les significations indiquées pour la formule I dans la revendication 1;
b) on isole le diclavulanate d'alkylène diammonium ainsi obtenu, de formule I 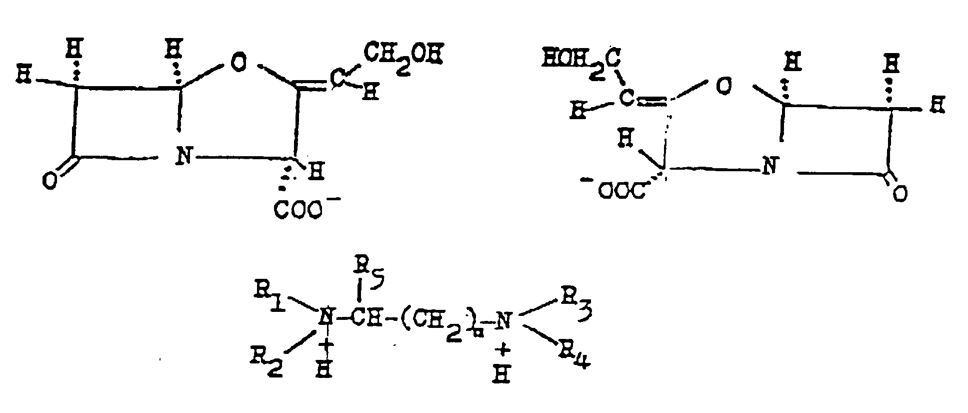 dans laquelle les substituants R₁, R₂, R₃, R₄ et R₅ ainsi que n ont les significations indiquées pour la formule I dans la revendication 1; et
c) on le convertit ensuite en acide clavulanique pur ou en des sels pharmaceutiquement acceptables de celui-ci.

12. Procédé selon la revendication 9 ou la revendication 11, caractérisé en ce que le clavulanate de potassium pur est fabriqué en faisant réagir du diclavulanate d'alkylène diammonium de formule I avec du 2-éthyl-hexanoate de potassium dans de l'isopropanol.

13. Procédé selon l'une quelconque des revendications 9, 11 ou 12, caractérisé en ce que le solvant organique est choisi dans le groupe comprenant l'acétate d'éthyle, la méthylisobutylcétone, le butanol ou un mélange d'acétate d'éthyle et d'acétone, d'éthylméthylcétone ou de méthylisobutylcétone.

14. Procédé selon la revendication 13, caractérisé en ce que le solvant organique utilisé est l'acétate d'éthyle.

15. Procédé de fabrication de clavulanate de potassium pur, caractérisé en ce que
a) on fait réagir de l'extrait d'acide clavulanique brut dans de l'acétate d'éthyle avec de la N,N'-diisopropyléthylène diamine,
b) on isole le diclavulanate de N,N'-diisopropyléthylène diammonium ainsi obtenu, et
c) on le fait ensuite réagir avec du 2-éthyl-hexanoate de potassium dans de l'eau contenant de l'isopropanol pour le convertir en clavulanate de potassium pur et on isole le sel souhaité.
